# EUROPEAN PATENT APPLICATION

(11) **EP 1 029 539 A1**
(43) Date of publication of application: **23.08.2000**
(21) Application number: 00301271.3
(22) Date of filing: 17.02.2000
(51) Int. Cl.: A61K 9/48

(54) **Pharmaceutical filled hard capsules**

(30) Priority: 17.02.1999 JP 3849099
(71) Applicant: Shionogi Qualicaps Co., Ltd., Yamatokoriyama-shi, Nara-ken (JP); Gattefosse S.A., 69804 Saint Priest Cedex (FR)
(72) Inventor: Nagata, Shunji, Ashiya-shi, Hyogo-ken (JP); Hoshi, Noboru, Iruma-shi, Saitama-ken (JP)
(74) Representative: Stuart, Ian Alexander

(57) **Abstract**

A hard capsule shell formed mainly of a polysaccharide is filled with a pharmaceutical self-emulsifiable preparation, giving a filled hard capsule. The filled capsule is unsusceptible to shell cracking and remains stable in quality.

## Description

This invention relates to pharmaceutical filled hard capsules comprising a hard capsule shell filled with a pharmaceutical self-emulsifiable preparation.

Many active or pharmaceutical ingredients of drugs are poorly soluble in water and tend to raise a problem of difficult absorption through the digestive system. It is known that the absorption of pharmaceutical ingredients is retarded or increased by dissolving or dispersing the pharmaceutical ingredients in fats, oils or fatty acid derivatives (Pharmaceutical Technology Europe, vol. 10, No. 10, 1998).

Also known in the art are pharmaceutical self-emulsifiable preparations which immediately emulsify upon contact with water. These preparations contain pharmaceutical ingredients dissolved or dispersed in oil or fat components. Upon oral administration, the oil or fat component immediately forms a micro-emulsion in the digestive system so that the pharmaceutical ingredients are quickly absorbed in the body. Such self-emulsifiable preparations include a combination of a medium-chain fatty acid triglyceride, lecithin, and bile acid, and a combination of a saturated polyglycolated glyceride obtained by polyglycolysis of hydrogenated vegetable oil with polyethylene glycol, a mono-, di- or triglyceride, and a mono- or di-fatty acid ester of polyethylene glycol.

These self-emulsifiable preparations are known to be unstable when enclosed in capsules. More specifically, hard gelatin capsule shells are filled with self-emulsifiable preparations to form capsules. Since the self-emulsifiable preparations are highly hygroscopic, the hard gelatin capsule shells are deprived of water with the lapse of time. The hard gelatin capsule shells eventually crack, which is a serious drawback.

One typical means for manufacturing oils and fats into a dosage form is to fill soft or hard capsules therewith. Since soft or elastic capsules are of a relatively thick gage, the available volume relative to the overall volume of the capsule is small as compared with hard capsules. The number of capsules to be administered increases when a drug is to be administered in a large dose. On the other hand, hard capsules are of a relatively thin gage so that the shape and mechanical strength of the capsule are affected by the fill. Specifically, the hard gelatin capsules are readily affected by the temperature and moisture absorption of the fill. If the fill is hygroscopic, it is difficult to insure the stability of capsules during an accelerated test.

In order that a pharmaceutical ingredient-containing self-emulsifiable preparation be commercially manufactured into tablets, experimental and trial tablet manufacture is carried out at an early stage. A small amount of the preparation is manufactured at the experimental and trial stage. The process is then scaled up to comply with the commercial mass manufacture. At this point, manufacturing conditions including a compression speed and pressure must be precisely adjusted one by one before good tablets can be manufactured. Even when such adjustments are made, the performance of the resulting tablets, especially dissolution of the pharmaceutical ingredient varies among the tablets. It is then difficult to manufacture uniform tablets in an efficient manner.

It would be advantageous to provide a hard capsule comprising a hard capsule shell filled with a pharmaceutical self-emulsifiable preparation, which prevented the capsule shell from cracking and remains stable in quality.

The inventors have found that when a hard capsule shell formed mainly of a polysaccharide such as a water-soluble cellulose derivative is filled with a pharmaceutical self-emulsifiable preparation, the filled hard capsule is effective for preventing the capsule shell from cracking and maintains stable quality for a long period of time. Such capsules can be easily prepared regardless of manufacturing quantity, that is, from the small-quantity manufacture on the experimental and trial stage to the mass-scale manufacture.

The present invention provides a hard capsule comprising a hard capsule shell filled with a pharmaceutical self-emulsifiable preparation wherein the hard capsule shell is formed mainly of a polysaccharide.

### BRIEF DESCRIPTION OF THE DRAWINGS

FIG. 1 schematically illustrates a drop-weight impact tester for testing empty capsules.
FIG. 2 is a graph showing the percent breakage versus the water content of capsules.

### DESCRIPTION OF THE PREFERRED EMBODIMENTS

The pharmaceutical filled capsule of the invention preferably uses a non-gelatin hard capsule shell formed mainly of a polysaccharide.

The polysaccharides include water-soluble cellulose derivatives, pullulan, hemicellulose, corn starch, and chitosan. Exemplary water-soluble cellulose derivatives are carboxymethyl cellulose and water-soluble salts thereof such as sodium salts, and preferably cellulose esters in which some or all of the hydroxyl groups are replaced by alkyl groups, especially lower alkyl groups and/or hydroxy lower-alkyl groups. Illustrative examples are hydroxypropyl methyl cellulose, hydroxyethyl cellulose, hydroxypropyl cellulose, and hydroxyethyl methyl cellulose, with the hydroxypropyl methyl cellulose being most preferred for film-forming ability and mechanical strength at low water contents.

The hard capsule shell used herein may be formed by gelling the polysaccharide with a gelling agent. A proper gelling agent may be selected depending on the type of polysaccharide. Exemplary gelling agents are kappa-carrageenan, iota-carrageenan, lambda-carrageenan, tamarind seed polysaccharides, pectin, curdlan, gelatin, furcelleran, agar, xanthane gum, locust bean gum, and gellan gum. Specifically, carrageenan and gellan gum are preferred for cellulose ethers such as hydroxypropyl methyl cellulose. Xanthane gum, locust bean gum, gellan gum, and carrageenan are preferred for pullulan.

If necessary, a gelling assistant is used. Appropriate gelling assistants are water-soluble compounds containing at least one of a potassium ion, ammonium ion and calcium ion, for example, potassium chloride, potassium phosphate, calcium chloride and ammonium chloride for kappa-carrageenan; and water-soluble compounds containing a calcium ion, for example, calcium chloride for iota-carrageenan.

A process for preparing hard capsule shells using the gelling agent is described. Hard capsules shells are prepared by the same process as a conventional hard gelatin capsule forming process, that is, by dissolving the polysaccharide, the gelling agent, and optionally the gelling assistant in water to form an aqueous solution (or gel), dipping mold pins in the solution, pulling up the pins, causing the polysaccharide to gelatinize, and drying.

It is noted that JP-A 3-279325 discloses hard capsule shells based on water-soluble cellulose derivatives such as hydroxypropyl methyl cellulose to which a gelling agent is added.

Like conventional hard gelatin capsules, colorants such as dyes and pigments, opacifying agents, flavors, etc. may be added to the capsule shells used herein insofar as the objects of the invention are not impaired.

The hard capsule shell prepared by the above process preferably contains 65 to 98% by weight, especially 80 to 95% by weight of the polysaccharide as the main component, up to 10% by weight, especially up to 5% by weight of the gelling agent, and up to 10% by weight, especially up to 5% by weight of the gelling assistant, and has a water content of 2 to 15% by weight, especially 3 to 7% by weight. In some cases, the gelling agent and gelling assistant are omitted. A shell with a too low water content would be susceptible to crack whereas a shell with a too high water content would be weak.

The pharmaceutical self-emulsifiable preparation with which the hard capsule shell is internally filled is a preparation which is self emulsifiable, that is, reacts with a liquid, typically water to form an emulsion. Any of well-known self-emulsifiable preparations may be used. That is, any desired preparation may be used as long as it forms a self micro-emulsion upon contact with body fluid to promote dissolution and absorption of the pharmaceutical ingredient and assists the pharmaceutical ingredient in reaching the desired human tissue. Especially preferred is a self-emulsifiable preparation of the type that forms a self micro-emulsion upon contact with body fluid or water at a temperature approximate to the body temperature.

Known self-emulsifiable preparations include a composition comprising an ester of polyethylene glycol (containing a mixture of glyceride and a fatty acid ester and a co-surfactant) as described in JP-A 8-277215; a composition comprising a medium-chain fatty acid triglyceride, lecithin, and bile acid; and a composition comprising polyethylene glycol (400), water, a medium-chain fatty acid triglyceride, and a surfactant as described in Yakubutsu Dotai (Pharmaceutical Dynamics), vol. 13, No. 5, 516-523, 1998). Useful self-emulsifiable preparations include a formulation comprising an ester of polyethylene glycol as a main component, a formulation comprising polyethylene glycol (400), a medium-chain fatty acid triglyceride, and a surfactant, and a formulation comprising a lipophilic phase consisting of a mixture of a glyceride and a fatty acid ester, a surfactant, a co-surfactant, and a hydrophilic phase although the invention is not limited thereto.

The surfactant used herein is a compound having a hydrophobic long-chain aliphatic group and a highly hydrophilic polar or ionic group. Included are saturated polyglycol glycerides (monoglycerides, diglycerides, triglycerides, a mixture of polyethylene glycol monoester and diester, and a mixture of glycerin and free polyethylene glycol) and polyglycerin oleic esters. The co-surfactant used herein is a highly hydrophobic agent capable of inducing mutual dissolution between aqueous and oily phases of a micro-emulsion, for example, propylene glycol lauryl ester, polyglycerin oleic ester or diglycol ethyl.

Exemplary formulations of the self-emulsifiable preparation used herein include a combination of a medium-chain fatty acid triglyceride with lecithin and bile acid; a combination of a saturated polyglycolated glyceride obtained by polyglycolysis of hydrogenated vegetable oil with polyethylene glycol, with a mono-, di- or triglyceride and a mono- or di-fatty acid ester of polyethylene glycol; and a formulation comprising a lipophilic phase consisting of a mixture of a glyceride and a fatty acid ester, a surfactant in the form of a saturated polyglycol glyceride or polyglycerin oleic ester, and a co-surfactant in the form of propylene glycol lauryl ester, polyglycerin oleic ester or diglycol ethyl.

The pharmaceutical ingredient in the self-emulsifiable preparation is preferably a difficultly soluble one. Exemplary drugs include vitamins, antipyretic, analgesic, anti-inflammatory, anti-ulcer, cardiac agents, anticoagulants, hemostats, bone resorption suppressors, vascularization suppressors, antidepressants, antitumor agents, antitussive expectorants, muscle relaxants, antiepileptic agents, antiallergic agents, antiarrhythmic agents, vasodilators, hypotensive diuretic agents, antidiabetic agents, antitubercular agents, hormone agents, narcotic antagonistic agents, immunosuppressors, and anibacterial agents, especially those agents which are difficultly soluble in water. Besides the drugs, there may be used medicaments for animal and plant use as well as chemical ingredients in cosmetic and food fields.

The filled capsule of the invention is prepared by a conventional capsule filling method, that is, by mounting a hard capsule (consisting of a pair of body and cap parts) on a capsule filling machine, separating the body and the cap, charging the body with a predetermined dose of the self-emulsifiable preparation, and joining the cap to the body. If desired, a suitable component such as the capsule base is applied to the body-cap joint to form a band seal.

The filled hard capsule of the invention is effective for preventing the capsule shell from cracking or breaking with the lapse of time because the self-emulsifiable preparation within the capsule shell is maintained stable over a long period of time.

The capsules filled with the self-emulsifiable preparation represent a system which is advantageous in time and economy for the development of new drugs. More particularly, for the development of new drugs, time and economy are two important factors that have long been considered. In accordance with the recent innovation of the drug developing process, the number of candidate compounds is now several hundred or several thousand times greater than in the past. To accommodate such a great number of candidate compounds, the screening method is improved. However, an appropriate preparation or dosage form has not been devised (see Pharm-Tech-Japan, Vol. 14, No. 6, 827-831, 1998). At an early stage of development, one compound is furnished in a small amount. The desired dosage form at this stage is the one which can be prepared from a small amount and which are not affected by scaling up. One typical dosage form is tablets which are manufactured by a compression step. It is well known that the porosity of compressed tablets is dictated by the speed of the tablet machine. For those tablets to be administered to small animals such as rats and those tablets to be administered to humans, the compression pressure must be changed. For these reasons, the tablets are not identical in performance, especially the dissolution of the pharmaceutical ingredient.

In contrast, the filled capsules of the invention are insensitive to scaling-up because the pharmaceutical ingredient is supplied in the form fully dissolved or dispersed in the preparation. There have been developed small capsules that can be administered to small animals such as rats. For both small animals and humans, the filled capsules from small to large size can be manufactured without changing the composition of the capsule shell and without changing the formulation of the pharmaceutical preparation.

### EXAMPLE

Examples of the invention are given below by way of illustration and not by way of limitation.

### Experiment

There were furnished hydroxypropyl methyl cellulose (HPMC) capsules of the following composition.

| | |
|---|---|
| HPMC | 98.5 parts by weight |
| kappa-carrageenan | 1.0 part by weight |
| Potassium chloride | 0.5 part by weight |

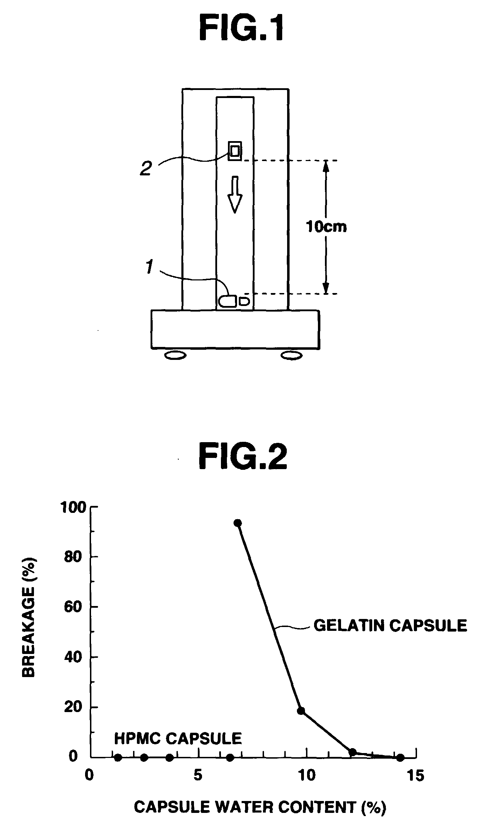

For comparison, gelatin capsules were also furnished. These empty capsules were conditioned in an environment at a controlled relative humidity. The water content of the capsules was measured by Karl Fischer method. Immediately after the water content measurement, the capsules were subjected to an impact test. Using a drop-weight impact tester as shown in FIG. 1, a weight 2 of 50 grams was dropped onto a capsule 1 from a height of 10 cm. For each type, fifty (50) capsules were tested, from which a percent of broken capsules was calculated.

FIG. 2 is a plot of the percent breakage versus the water content of capsules. HPMC capsules are tough. Note that all the capsules are of "1" size.

### Example 1 & Comparative Example 1

HPMC capsules each were filled with about 400 mg of Gelucire 44/14 (trade name, Gattefosse Corp.) which had been kept at 65°C. For comparison purposes, gelatin capsules each were filled with about 400 mg of Gelucire 44/14 which had been kept at 65°C. Note that all the capsules are of "1" size.

### Example 2 & Comparative Example 2

HPMC capsules each were filled with about 400 mg of Gelucire 50/13 (trade name, Gattefosse) which had been kept at 65°C. For comparison purposes, gelatin capsules each were filled with about 400 mg of Gelucire 50/13 which had been kept at 65°C. Note that all the capsules are of "1" size.

### Example 3 & Comparative Example 3

Polyethylene glycol (400), medium-chain fatty acid triglyceride (trade name, Miglyol 810), water and polyoxyethylene (20) cetyl ether (trade name, BC-20TX) were mixed in a weight ratio of 73.95 : 10.00 : 13.05 : 3.00 and kept at 65°C. HPMC capsules each were filled with about 400 mg of the composition. For comparison purposes, gelatin capsules each were filled with about 400 mg of the same composition kept at 65°C. Note that all the capsules are of "1" size.

### Example 4 & Comparative Example 4

Polyethylene glycol (400), medium-chain fatty acid triglyceride (trade name, Miglyol 810), water and polyoxyethylene (20) cetyl ether (trade name, BC-20TX) were mixed in a weight ratio of 73.95 : 10.00 : 13.05 : 3.00 and kept at about 50°C. HPMC capsules each were filled with about 400 mg of the composition. For comparison purposes, gelatin capsules each were filled with about 400 mg of the same composition kept at about 50°C. Note that all the capsules are of "1" size.

### Example 5 & Comparative Example 5

HPMC capsules each were filled with about 400 mg of Gelucire 44/14 (trade name, Gattefosse) which had been kept at about 50°C. For comparison purposes, gelatin capsules each were filled with about 400 mg of Gelucire 44/14 which had been kept at about 50°C. Note that all the capsules are of "1" size.

### Example 6 & Comparative Example 6

HPMC capsules each were filled with about 400 mg of Gelucire 50/13 (trade name, Gattefosse) which had been kept at about 50°C. For comparison purposes, gelatin capsules each were filled with about 400 mg of Gelucire 50/13 which had been kept at about 50°C. Note that all the capsules are of "1" size.

### Test 1

The filled capsules of Examples 1-3 and Comparative Examples 1-3 were examined by a finger clamping test as prepared and after they were stored for one day at 60°C. The capsule was rated "OK" when the capsule did not crack by lightly clamping between thumb and forefinger and "Fail" when the capsule cracked by lightly clamping between thumb and forefinger. The results are shown in Table 1.

**Table 1**

| | Capsule shell | As prepared | Aged 60°C, 1 day |
|---|---|---|---|
| Example 1 | HPMC | OK | OK |
| CE 1 | gelatin | OK | Fail |
| Example 2 | HPMC | OK | OK |
| CE 2 | gelatin | OK | Fail |
| Example 3 | HPMC | OK | OK |
| CE 3 | gelatin | OK | Fail |

### Test 2

The filled capsules of Examples 4-6 and Comparative Examples 4-6 were examined by a finger clamping test as prepared and after they were stored for one month at 45°C. The capsules were emptied of the contents before the test. The capsule was rated "OK" when the capsule did not crack by lightly clamping between thumb and forefinger; "Moderate" when the capsule did not crack, but somewhat changed its outer appearance by lightly clamping between thumb and forefinger; and "Fail" when the capsule cracked by lightly clamping between thumb and forefinger. The results are shown in Table 2.

**Table 2**

| | Capsule shell | As prepared | Aged 45°C, 1 month |
|---|---|---|---|
| Example 4 | HPMC | OK | Moderate |
| CE 4 | gelatin | OK | Fail |
| Example 5 | HPMC | OK | OK |
| CE 5 | gelatin | OK | Fail |
| Example 6 | HPMC | OK | OK |
| CE 6 | gelatin | OK | Fail |

After the filled capsules of Examples 4-6 and Comparative Examples 4-6 were stored for one month at 45°C, the capsules were emptied of the contents. Three empty capsules for each type were measured for mechanical strength using the impact tester shown in FIG. 1. The number of broken capsules is reported in Table 3.

**Table 3**

| | Capsule shell | Aged 45°C, 1 month |
|---|---|---|
| Example 4 | HPMC | 0/3 |
| CE 4 | gelatin | 3/3 |
| Example 5 | HPMC | 0/3 |
| CE 5 | gelatin | 2/3 |
| Example 6 | HPMC | 0/3 |
| CE 6 | gelatin | 2/3 |

The pharmaceutical filled hard capsules of the invention, even though they are filled with a pharmaceutical self-emulsifiable preparation, do not invite shell cracking and remain stable in quality.

## Claims

1. A filled hard capsule comprising a hard capsule shell filled with a pharmaceutical self-emulsifiable preparation, characterized in that said hard capsule shell is formed mainly of a polysaccharide.

2. The filled hard capsule of claim 1 wherein said self-emulsifiable preparation contains a polyethylene glycol ester as a main component.

3. The filled hard capsule of claim 1 wherein said self-emulsifiable preparation contains polyethylene glycol (400), a medium-chain fatty acid triglyceride, and a surfactant.

4. The filled hard capsule of claim 1 wherein said self-emulsifiable preparation contains a lipophilic phase consisting of a mixture of a glyceride and a fatty acid ester, a surfactant, a co-surfactant, and a hydrophilic phase.

5. The filled hard capsule of claim 1 wherein said polysaccharide is a water-soluble cellulose derivative.

6. A capsule according to any preceding claim wherein said hard capsule shell consists essentially of polysaccharide optionally together with one or more of: gelling agent, water and gelling assistant as structural components; and optionally one or more non-structural components such as colorants, opacifying agents and flavors.

7. A capsule according to any preceding claim wherein said hard capsule shell contains, by weight, 65-98% of polysaccharide, 0-10% of gelling agent, 0-10% of gelling assistant and 2-15% of water.

8. A capsule according to Claim 7 wherein said shell contains, by weight, 80-95% of polysaccharide, 0-5% of gelling agent, 0-5% of gelling assistant, and 3-7% of water.

9. A hard capsule formed mainly of a polysaccharide and suitable for use in forming a filled hard capsule according to any preceding claim.
